# EUROPEAN PATENT APPLICATION

(11) **EP 2 754 464 A1**
(43) Date of publication of application: **16.07.2014**
(21) Application number: 13305037.7
(22) Date of filing: 15.01.2013
(51) Int. Cl.: A61N 1/38, A61N 1/372

(54) **Implantable electronic device**

(71) Applicant: Fontaine, Guy, 94160 Saint Mande (FR)
(72) Inventor: Fontaine, Guy, 94160 Saint Mande (FR)
(74) Representative: Hepp Wenger Ryffel AG

(57) **Abstract**

An implantable electronic device for delivering electric shocks to intentionally induce a fibrillation comprises at least one electrode (2) for application on the body. The electrode (2) is in particular applied on the right ventricle. The device (1) further comprises a main electronic unit (3) for controlling operation of the device (1) and an isolated wire (4) between the main electronic unit (3) and the electrode (4). The main electronic unit (3) is adapted to generate an electric shock capable of inducing fibrillation. The main electronic unit further comprises means for deploying an electric shock (5) adapted to induce a fibrillation, wherein the means for deploying an electric shock (5) are programmable or deployable by the mammal carrying the device (1) in his body or a duly authorized person or organisation.

## Description

The invention is directed to an implantable electronic device for delivering electric shocks to intentionally induce a fibrillation according to the preamble of the independent claim.

Medically assisted suicide (euthanasia) has been accepted by a wide fraction of the population. In countries such as Switzerland euthanasia is provided e.g. by associations. In France, according to recent polls, 80% of the inhabitants are favourable to euthanasia. Furthermore, euthanasia is practiced frequently with mammals such as pets which suffer from disease or pain and for which no treatment is available or possible. However, bringing pets to a veterinarian doctor for this purpose may cause unnecessary stress to the mammal and/or to the person responsible for the mammal.

It is well known to enable mammals to die by means of toxic substances, which is for example disclosed for the case of humans in DE 299 20 843 U1, where humans being buried alive are equipped with a toxic substance to enable them taking their life. This method is not reliable, since due to the physical differences, some people may survive the intoxication, having damaged their health or maybe suffer over a long time until they die.

From EP 0 516 811 a composition is known, which leads to a humane euthanasia of a mammal. This composition has to be applied by physician and can not be used by person himself.

In the state of the art defibrillators are known, which are implanted inside the body of a patient. In case of an occurring fibrillation, the defibrillator induces electric shocks into the heart to stop the fibrillation and re-create a normal heart rhythm. Such a device is for example disclosed in EP 0 773 039. Due to the shape of the wave of the electric shock, this device is suited for intentionally defibrillating the heart.

It is an object of the present invention to avoid the disadvantages of prior art and in particular to provide a device enabling a mammal or a person deciding for the mammal to voluntarily decide on the time and the way of death.

The object is accomplished by an implantable electronic device for delivering electric shocks to intentionally induce a fibrillation. The device comprises at least one electrode for application on the body, in particular the right ventricle, and a main electronic unit for controlling operation of the device. An isolated wire is arranged between the main electronic unit and the electrode. The main electronic unit is adapted to generate an electric shock having a form capable of inducing fibrillation. The main electronic unit further comprises means for deploying the electric shock adapted to induce a fibrillation, wherein the means for deploying the electric shock are programmable or deployable by the mammal carrying the device in his body or a duly authorized person or organisation.

Such a device enables a mammal to individually decide on the point of time where his life or the life of a mammal he is responsible for ends. At this point, the mammal does not need any help from any external person but is able to end life himself without endangering others physically or legally. Furthermore, the decision and the accomplishment can take place within the usual surroundings without any external people being present.

The electric shock can be deployed e.g. in form of sinus wave, preferably at 50 Hz or in form of bursts,e.g. a peak voltage, preferably a series of peak voltages. The peak voltages preferably have the shape of square impulses with a short interval between impulses such as 50 ms. The amplitude can be in the range of 4-8 volts such as conventional pacemakers. The device can test if fibrillation has been properly triggered after one or several attempts.

Such an electric shock leads to a fast creation of a fibrillation and hence to the occurrence of a fast death.

The device can comprise at least one additional sensor, preferably a sensor of inertia or a sensor of acceleration. Other possible sensors are temperature sensors, sensors for measuring impedance of the thorax, photoelectric sensors, pH sensors, endocavity sensors or others.

Additional sensors enable detection of outer conditions such as movement of the person or physical conditions. This way the measurement of the additional sensors can be taken into account when determining the time for deploying an electric shock or when detecting an illness that can be curable or not.

The temperature sensor can comprise two metals being connected, wherein a difference in potential is measurable depending on temperature. Preferably, the temperature sensor is arranged on the side of the device being on the far side of the skin, since the skin temperature is below the actual body temperature.

The sensor for impedance of the thorax preferably is formed by the main electronic unit and the electrode, wherein a weak current at a high frequency, e.g. 100 KHz, is created between the electrode and the main electronic unit and the impedance is measured.

The endocavity sensor can record a pseudo or a kind of ECG.

The photoelectric sensor is capable of measuring the partial content of carbon dioxide as known from the state of the art.

The pH sensor is capable of measuring the pH value of the blood, which in a healthy state corresponds to a pH-value of 7.

The acceleration sensor preferably comprises three single acceleration measurement parts, one for each dimension. Out of the measured values of the acceleration sensor a profile can be deduced which enables a conclusion on possible illnesses or status of the mammal wearing the device. Such illnesses could be neurological illnesses for example.

The means for deploying the electric shock can be programmable such that the electric shock is deployed immediately or such that the electric shock is deployed randomly within a pre-specified period of time or depending from certain external criteria. Preferably the means for deploying an electric shock are programmable from an external program device. The programming of the means for deploying an electric shock leads to either the specific definition of a point of time where the electric shock is applied or a time frame where the electric shock is applied. Hence, the person programming the device, preferably the person wearing the device inside his body or the person being responsible for a mammal, is able to determine when and where the electric shock is going to be deployed. The means for deploying an electric shock can also be design such as to be only activatable after predefined criteria are fulfilled. Preferably the predefined criteria are predefined values of the at least one additional sensor.

An enablement of the device only upon the fulfilment of predefined criteria allows defining situations or locations where the device is not activatable. Hence, the user can decide on situations where an activation of the device is not possible, such as certain times or places or physical conditions of the mammal carrying the device itself. Furthermore, it allows preventing deployment if the mammal wearing the device is located in specific areas such as crowded places or only allows deployment in a specific location such as the home during sleep or in laying position. The triggering of fibrillation preferably takes place nocturnal and in a laying position.

The main electronic unit can further be operable in a second mode of operation, wherein when a fibrillation of the heart occurs without fulfilling the predefined criteria, an electric shock is generated to induce a defibrillation.

Hence, a user can decide on situations or places or times according to the predefined criteria where he further wants the main electronic unit to conduct a defibrillation, hence, to save life. In this case the main electronic unit deploys an electric shock preferably in form of a bi-phasic defibrillation wave, as known from the state of the art.

Furthermore, the device can be operable in a third mode of operation, wherein the device is inactivated. In such a state the connection between a power source and the device is disconnected and can be reconnected by magnetic or electromagnetic intervention. A magnetic intervention or electromagnetic intervention can for example be realized by a Reed-Switch within the device between power source and main electronic unit. Such a state enables the implantation of the device into a mammal well before there is any need for using the device.

In a fourth state the device is blocked. This can for example be the case if a malfunction has been detected, by the device itself or by a Centre of Decision. Such a state obviates a malfunction e.g. a wrong deployment of an electric shock. The system can also be designed for carrying out automatic self tests.

The main electronic unit can comprise a wireless communication device, preferably a mobile phone communication device such as a GSM or UMTS or LTE or other mobile phone standard device and/or a GPS emitter and/or receiver and/or a WiFi connection unit.

Instead of GPS of course other satellite navigation and localization systems can be used such as the Galileo system.

A wireless communication device enables the localization of the carrier of the device and can be used to inform organ donation organisations or funeral parlours of the position and the time of death of the person wearing the device. Also, it allows preventing deployment if the person wearing the device is located in specific areas such as crowded spaces or only allows deployment in a specific location such as the home. Furthermore, the wireless communication device allows communication of the device with a Centre of Decision or any other adapted communication device. Hence, the device can further send information or be controlled or programmed wirelessly. For example the state of the device, such as inactivated, activated or blocked can be set wirelessly.

The means for deploying an electric shock can be synchronizable with means for deploying an electric shock of a second device.

The synchronizing means lead to the possibility of inducing death into two different persons at the same point of time. This could be desirable for couples, siblings or twins, for example.

The device can further comprise means for communication with a centre of decision.

A centre of decision can collect for example predefined criteria and signatures or documents of the mammal wearing the device. The main duty of the centre of decision is the surveillance of the mammal wearing the device by the integrated sensors as described above. The centre of decision can further be applied for detecting illnesses without the need of consulting a doctor or hospital, based on the values detected by the sensors as described before. Additionally, the centre of decision can be connected to a medical library, in which all information regarding the mammal wearing the device is stored. Furthermore, even administrative data could be stored in a library and be accessible for the Centre of Decision. Hence, in case the mammal wearing the device dies, all data can easily be transferred to wherever needed.

The device can further comprise means for informing a third person or organisation about the time of deployment of the electric shock. Preferably this information is delivered to the third person or organisation in advance of the deployment of the electric shock.

Hence, a third person can be informed of the time of death in advance and by this be prepared of the death of the mammal wearing the device. Where legally allowed, this third person can also decide for a person wearing the device, in case the person is seriously ill with no hope for recreation and not anymore in a position to make a decision. Furthermore, an organisation or institution for organ donation may be informed in advance, to enable instantaneous removal of the organs needed to ensure that the organs still can be implanted into other people.

The device can further comprise means for activation of the device, preferably by an activation code.

Additionally, the device can comprise means for deactivation of the device, preferably by a deactivation code.

The means for activation of the device enable the implementation of the device well in advance if the person wearing the device or the person deciding for a mammal wishes this. In case the device is implanted in advance, the device can not be deployed unless it is purposely activated by the person wearing the device or the person being responsible for a mammal.

The power source of the device can be a battery, preferably a lithium battery having a long lifetime.

All materials of the device are preferably biocompatible, such as titanium.

The deployment of the device can be dependent on an activity level of the mammal wearing the device.

Such a possibility leads to prevention of long suffering of the mammal wearing the device. Euthanasia is considered today as an alternative, only after long periods of suffering. With the present invention the beginning of such periods suffering may be detected in advance, e.g. based on activity detection. If the activity is below a certain threshold the device may be activated or deployment may be made possible - leading to a medically assisted suicide.

The components are basically formed in a manner known for implantable defibrillators.

The invention is further explained with reference to preferred embodiments and the following figures which show:
- Figure 1: A structural overview of the device.
- Figure 2: A preferred waveform of the electric shock.

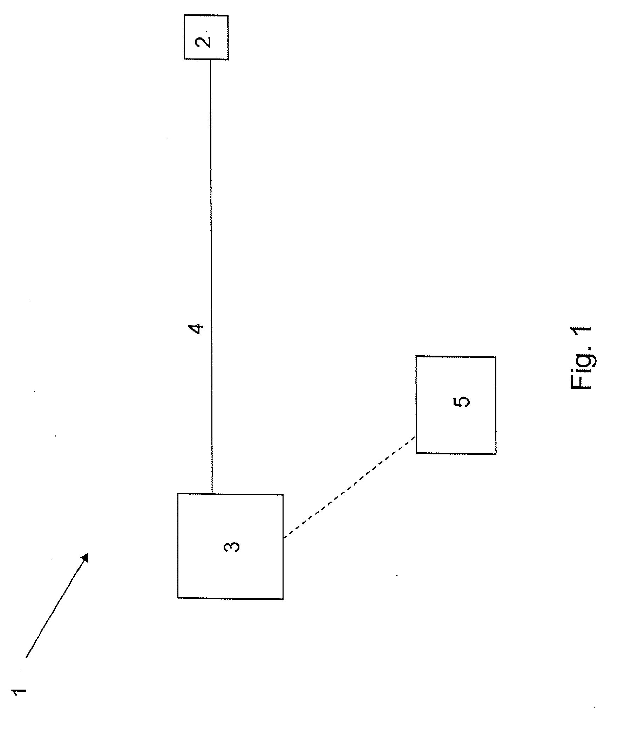

Figure 1 shows an implantable electronic device 1 comprising an electrode 2 and a main electronic unit 3. The electrode 2 and the main electronic unit 3 are connected by an isolated wire 4. The main electronic unit 3 controls the operation of the device 1 and is adapted to generate an electric shock capable of inducing fibrillation in a mammal. The device 1 further comprises means for deploying an electric shock 5, which are programmable or deployable by the mammal carrying the device in his body or a duly authorized person or organisation. The components are basically formed in a manner used by skilled persons for implacable defibrillators.

The means for deploying an electric shock 5 can be programmed to immediately or within a certain time frame induce an electric shock by the main electronic unit 3 over the isolated wire 4 and the electrode 2 directly into the heart of the person wearing the device. The means for deploying an electric shock 5 are connected to the main electronic unit 3 wirelessly by known protocols such as Bluetooth. Furthermore, the means for deploying an electric shock 5 can be coupled to a centre of decision (not shown), where physical parameters or other parameters are stored and used as decision criteria for the possibility of applying an electric shock. The main electronic 3 unit may further be provided with additional sensors such as sensors for measurement of inertia or sensors for measurement of acceleration. The main electronic unit additionally may comprise a GPS emitter and/or a GSM device for tracking the position of the mammal carrying the implantable device 1 and communication with the main electronic unit 3 wirelessly. The electrode 2 is configured such as being capable of measuring the heart's contractions and of deploying an electric shock.

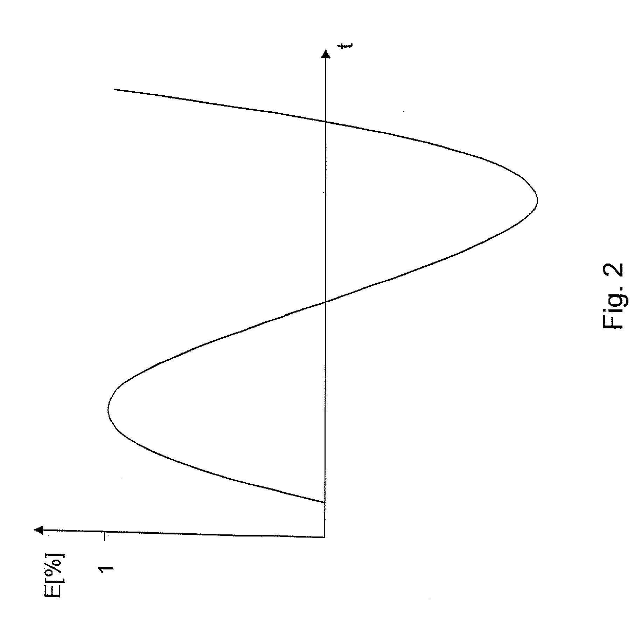

Figure 2 shows the preferred waveform of the electric shock deployed by main electronic unit 3 (see figure 1) to induce a fibrillation. The wave form is a sinus-wave of the energy in percent over time.

### References

- 1: Device
- 2: Electrode
- 3: Main electronic unit
- 4: Isolated wire
- 5: Means for deploying an electric shock

## Claims

1. Implantable electronic device (1) for delivering electric shocks to intentionally induce a fibrillation comprising at least one electrode (2) for application on the body, in particular the right ventricle, a main electronic unit (3) for controlling operation of the device, an isolated wire (4) between the main electronic unit (3) and the electrode (2), wherein the main electronic unit (3) is adapted to generate an electric shock having a form capable of inducing fibrillation, the unit further comprising means for deploying an electric shock (5) adapted to induce a fibrillation, wherein the means for deploying the electric shock (5) are programmable or deployable by the mammal carrying the device in his body or a duly authorized person or organization.

2. Device (1) according to claim 1, **characterized in that** the electric shock is deployed in form of a sinus wave, preferably at 50 Hz

3. Device (1) according to any one of the preceding claims, **characterized in that** the device (1) comprises at least one additional sensor, preferably a sensor of inertia or a sensor of acceleration.

4. Device (1) according to any one of the preceeding claims, **characterized in that** the means for deploying an electric shock (5) are programmable such that the electric shock is deployed immediately or such that the electric shock is deployed randomly within a pre-specified period of time, preferably programmable from an external programming device.

5. Device (1) according to any one of the preceding claims, **characterized in that** the means for deploying an electric shock (5) are only activatable after predefined criteria are fulfilled, preferably predefined values of the at least one additional sensor.

6. Device (1) according to claim 5, **characterized in that** the main electronic unit (3) is operable in a second mode of operation, wherein when a fibrillation occurs without fulfilling the predefined criteria an electric shock is generated to induce a defibrillation.

7. Device (1) according to any one of the preceding claims, **characterized in that** the main electronic unit (3) comprises a wireless communication device, preferably a mobile phone communication device such as a GSM or UMTS or LTE device and/or a GPS emitter and/or receiver.

8. Device (1) according to any one of the preceding claims, **characterized in that** the means for deploying an electric shock (5) are synchronizable with means for deploying an electric shock (5) on a second device.

9. Device (1) according to any one of the preceding claims, **characterized in that** the device (1) further comprises means for communication with a Centre of Decision.

10. Device (1) according to any one of the preceding claims, **characterized in that** the device (1) further comprises means for informing a third person or organization about the time of deployment of the electric shock, preferably in advance.

11. Device (1) according to any one of the preceding claims, **characterized in that** the device (1) further comprises means for activation or deactivation of the device (1), preferably by an activation code.
